# EUROPÄISCHE PATENTSCHRIFT

(11) **EP 3 319 674 B1**
(45) Veröffentlichungstag und Bekanntmachung des Hinweises auf die Patenterteilung: **17.02.2021**
(21) Anmeldenummer: 16734187.4
(22) Anmeldetag: 24.06.2016
(51) Int. Cl.: A61M 15/08

(54) **NASENAPPLIKATOR**
NASAL APPLICATOR
APPLICATEUR NASAL

(30) Priorität: 06.07.2015 DE 202015004852 U
(43) Veröffentlichungstag der Anmeldung: 16.05.2018
(73) Patentinhaber: 5med GmbH, 82031 Grünwald (DE)
(72) Erfinder: HIERONYMUS, Jens, 64297 Darmstadt (DE); SINNER, Klaus, 80807 München (DE); NARDI-HIEBL, Stefan, London W1U 5LP (GB)
(74) Vertreter: Thoma, Michael
(86) Internationale Anmeldenummer: PCT/EP2016/001083
(87) Internationale Veröffentlichungsnummer: WO 2017/005344

(56) Entgegenhaltungen:
- EP-A2- 1 293 224
- WO-A1-2016/100564
- WO-A2-2007/081947
- DE-U1-202012 008 892

## Beschreibung

Die vorliegende Erfindung betrifft einen Nasenapplikator zum nasalen Verabreichen von medizinischen Wirkstoffen, insbesondere Schmerzmittel, mit einem in ein Nasenloch einführbaren Abgabedorn, einem in einem Applikatorgehäuse angeordneten verbindbaren Wirkstoffspeicher sowie einem Abgabedosierer zum dosierten Abgeben des Wirkstoffs aus dem Wirkstoffspeicher über den Abgabedorn.

In der Schmerztherapie müssen bisweilen starke Schmerzmittel bzw. Opioide verabreicht werden, wobei es, um einen schnellen Wirkeintritt zu erreichen, vorteilhaft ist, diese Schmerzmittel über die Nasenschleimhaut zu verabreichen. Hierfür vorgesehene Nasenapplikatoren sind in verschiedenen Formen bekannt und können vom Patienten selbst in einfacher Weise bedient werden. Der Nasenapplikator wird mit seinem Abgabedorn in ein Nasenloch eingeführt und sodann betätigt, um eine vorbestimmte Dosis des Schmerzmittels beispielsweise in zerstäubter Form eines Aerosols abzugeben. Ein im Inneren des Nasenapplikators vorgesehener Abgabedosiermechanismus kann beispielsweise eine motorgetriebene Pumpe umfassen, ist bisweilen aber auch ein handbetätigter Fördermechanismus, der beispielsweise durch Zusammendrücken des Applikatorgehäuses oder Eindrücken eines Förderkolbens arbeiten kann.

Dabei wurde auch bereits angedacht, an sich herkömmliche Nasenspray-Kartuschen einzusetzen, die eine integrierte Förder- bzw. Pumpvorrichtung aufweisen, die durch Bewegen des Abgabedorns auf den Kartuschenbehälter zu betätigt werden kann, indem beispielsweise der Abgabedorn an zwei Halteflügeln mit zwei Fingern gehalten und die Kartusche mit dem Daumen auf den Abgabedorn gedrückt wird. Um eine unsachgemäße, insbesondere unberechtigte oder überdosierte Verwendung solcher an sich handelsüblicher Nasenspray-Kartuschen zu verhindern, können diese Nasenspray-Kartuschen in ein Applikatorgehäuse eingesetzt werden, um die Wirkstoffabgabe aus der Nasenspray-Kartusche wahlweise sperren oder freigeben und ggfs. auch die Bedienung erleichtern zu können, was insbesondere für gebrechliche oder entkräftete Patienten hilfreich sein kann. Die Nasenspray-Kartusche kann dabei mit ihrem Wirkstoffbehälter verschieblich im Applikatorgehäuse aufgenommen sein, während der Abgabedorn festgehalten oder an einem Anschlag anstehen kann, sodass durch Verschieben des Wirkstoffbehälters relativ zum Applikatorgehäuse die Fördereinrichtung der Nasenspray-Kartusche betätigt werden kann.

Hierzu kann am Applikatorgehäuse eine Bedientaste von Hand bewegbar angeordnet sein, beispielsweise in Form eines Betätigungshebels oder Betätigungshahns, der umfangsseitig am Applikatorgehäuse vorstehen und in radialer Richtung eindrückbar sein kann, sodass ähnlich einer natürlichen Greifbewegung durch Zusammendrücken der das Applikatorgehäuse greifenden Hand bzw. Faust eine Betätigung herbeigeführt werden kann. Grundsätzlich kann eine solche Bedientaste jedoch auch nach Art eines Schiebers oder einer linear eindrückbaren Drucktaste oder nach einer Art eines Wippschalters oder Drehschalters ausgeführt sein. Die Betätigungsstellbewegung der genannten Bedientaste kann dabei durch einen Übertrager auf den Abgabedosierer übertragen werden, der dann für den Wirkstoffaustrag sorgt.

Nasenapplikatoren der eingangs genannten Art sind beispielsweise aus den Schriften US 2005/0098583, FR 2 721 521, WO 2016/100564 A1, WO2017005344 oder GB 2 408 214 bekannt, wobei die letztgenannte GB 2 408 214 eine Sperrung der Wirkstoffabgabe bei unsachgemäßem Ansetzen des Applikators vorsieht. Die FR 2 721 521 zeigt einen Nasenapplikator für die nasale Pulverapplikation.

Während einerseits die Selbstverabreichung bei Schmerzmitteln sinnvoll und erwünscht ist, um das Schmerzmittel nur dann zu geben, wenn es tatsächlich benötigt wird, muss andererseits der sich das Schmerzmittel selbst verabreichende Patient vor einer unbeabsichtigten Überdosierung geschützt werden, insbesondere wenn es sich um stark wirkende Opioid-Schmerzmittel handelt.

Die Schrift DE 10 2013 012292 schlägt hierzu vor, an dem Nasenapplikator eine Dosiserfassungseinrichtung vorzusehen, mittels derer die abgegebene Wirkstoffmenge erfasst wird. Ist eine jeweils zulässige Höchstdosis erreicht, wird der Abgabedosierer gesperrt, sodass keine weiteren Hübe bzw. Dosen abgegeben werden können. Dabei kann die Höchstdosis patientenindividuell programmiert werden, beispielsweise in einem am Handgelenk des Patienten befestigbaren RFID-Chip, aus dem die Steuervorrichtung des Nasenapplikators die zulässige Höchstdosis ausliest und den Abgabedosierer entsprechend freischaltet oder sperrt.

Eine Schwierigkeit besteht bei solchen patientenautonom-bedienbaren Nasenapplikatoren dabei jedoch darin, dass die von einer elektronischen Steuervorrichtung ansteuerbaren Ventile und Förderpumpen relativ viel Strom und damit größere Ackus oder Batterien benötigen, die den Nasenapplikator schwer und unhandlich zu bedienen machen. Diesbezüglich kommt hinzu, dass der Applikator an sich dauerhaft einsatzbereit sein soll, ohne erst eingeschaltet und hochgefahren werden zu müssen, um einem Patienten bei Bedarf rasch und jederzeit eine Selbstverabreichung des Wirkstoffs zu ermöglichen. Eine entsprechende Standby-Schaltung erhöht den genannten Strombedarf jedoch weiter.

Zur Umgehung oder Abmilderung dieser Problematik wäre an sich eine mechanische Betätigung des Dosierförderers geeignet, die auf elektrische Pumpen und fremdenergiebetätigte Ventileinrichtungen verzichtet. Solche mechanischen Betätigungsvorrichtungen, die den Abgabedosierer per Muskelkraft betätigen, sind jedoch problematisch, was die exakte Dosierung und immer gleichbleibende, reproduzierbare Ausführung der Dosierhübe anbelangt, da die abgegebene Dosis schwanken kann, je nachdem, ob die Betätigung kräftig oder weniger kräftig, schnell oder langsam oder die Bedientaste weiter oder weniger weit bewegt wird.

Selbst wenn die Bedientaste vollständig durchgezogen bzw. durchgedrückt wird, sodass an sich der volle Stellweg mit dem ganzen damit einhergehenden Fördervolumen erzeugt wird, kann es bei zu langsamer und ggfs. auch bei zu schneller Betätigung oder nicht ausreichend druckvollen Betätigungen zu Beeinträchtigungen bzw. Abweichungen im Sprühbild kommen. Solche Abweichungen im Sprühbild können sich beispielsweise dadurch ergeben, dass bei zu langsamer Betätigung die Tröpfchen relativ groß bleiben bzw. der Wirkstoff nicht ausreichend fein zerstäubt wird, sodass die Wirkstoffaufnahme durch den Körper beeinträchtigt ist, da diese stark von der Tröpfchen- bzw. Partikelgröße abhängt. Hierbei kann prinzipiell auch eine zu kleine bzw. zu feine Zerstäubung hinderlich sein, da sich dann der Wirkstoff nicht mehr an den Nasenwandungen absetzt, sondern direkt eingeatmet wird, was je nach Wirkstoff wünschenswert oder nicht wünschenswert sein kann. Andererseits können Abweichungen im Sprühbild auch durch die Austragsgeschwindigkeit bedingt sein, die je nach Betätigungsstärke und -geschwindigkeit variieren kann, was sich dann beispielsweise in einem schmäleren, weiter tragenden oder umgekehrt breiteren, kürzer tragenden Sprühkegel niederschlagen kann und, je nachdem, verschiedene Bereiche des Naseninnenraums verschieden stark beaufschlagen kann, was wiederum Auswirkungen auf die Wirkstoffaufnahme haben kann. Gerade bei älteren oder schwächeren Patienten kann es zu größeren Abweichungen bei der Betätigungsgeschwindigkeiten und den Betätigungskräften kommen, die in der genannten Weise zu unerwünschten Abweichungen im Sprühbild führen.

Zum anderen ist es bei solchen mechanischen Betätigungsmechanismen auch nicht ganz einfach, eine Sperrung des Betätigers ausreichend manipulationssicher auszuführen, ohne den mechanischen Betätigungs- bzw. Antriebsstrang massiv und klobig auszubilden. Um zu verhindern, dass die Bedientaste - beispielsweise nach Erreichen der zulässigen Tageshöchstdosis - nicht mit Gewalt entgegen einer Sperre ein weiteres Mal niedergedrückt wird, müsste die Sperrklinke oder ein ähnliches Sperrelement ausreichend massiv ausgebildet werden und auch gegen Manipulation von außen beispielsweise durch einen Schraubenzieher oder eine Nadel durch eine massive Gehäuseausbildung geschützt werden. Dies steht jedoch wiederum einer leichten Geräteausbildung entgegen und beeinträchtigt eine einfache Handhabung.

Hiervon ausgehend liegt der vorliegenden Erfindung die Aufgabe zugrunde, einen verbesserten Nasenapplikator der eingangs genannten Art zu schaffen, der Nachteile des Standes der Technik vermeidet und letzteren in vorteilhafter Weise weiterbildet. Insbesondere soll eine präzise, einfach zu bedienende Selbstdosierung hochwirksamer Schmerzmittel ermöglicht werden, ohne hierfür einen unhandlichen, schweren Dosierförderer, der eine hohe Akkuleistung verbraucht, zu benötigen.

Erfindungsgemäß wird die genannte Aufgabe durch einen Nasenapplikator gemäß Anspruch 1 gelöst. Bevorzugte Ausgestaltungen der Erfindung sind Gegenstand der abhängigen Ansprüche.

Es wird also vorgeschlagen, der Betätigungstaste gegenüber dem Abgabedosierer einen Freilauf bzw. Freigang zu geben und nur ausnahmsweise, nämlich dann wenn ein berechtigter Dosierhub ausgeführt werden soll, eine Wirk- bzw. Antriebsverbindung zwischen der Betätigungstaste und dem Abgabedosierer herzustellen. Durch die im Normal- bzw. Ausgangszustand gekappte Wirkverbindung kann die Bedientaste wirkungslos niedergedrückt werden, ohne dass dies zur Betätigung des Abgabedosierers führen würde, sodass auf massive Sperrelemente zum Blockieren der Bedientaste verzichtet werden kann, während andererseits bei hergestellter Wirkverbindung eine einfach händische Bedienung erfolgen kann, die keine schweren Batterien oder Akkus erfordert. Erfindungsgemäß ist der Übertrager zwischen der Betätigungstaste und dem Abgabedosierer ein- und auskuppelbar ausgebildet, sodass bei ausgekuppeltem Übertrager die Betätigungstaste im Freigang ohne Übertragung auf den Abgabedosierer bewegbar ist, wobei ein Bewegungssensor zum Erfassen von Bewegungen der Bedientaste und ein Kupplungsschalter zum Einkuppeln des Übertragers in Abhängigkeit eines Signals des Bewegungssensors vorgesehen sind. Durch den Bewegungssensor erhält die Betätigungstaste eine Doppelfunktion, da sie einerseits zum Scharfstellen bzw. Aufwecken des Systems und andererseits zum Betätigen des Abgabedosierers verwendet werden kann. Wird die Bedientaste zunächst aus der Ausgangsstellung heraus betätigt, erfasst dies der Bewegungssensor und meldet dies dem Kupplungsschalter, der dann den Übertrager einkuppeln und damit den Betätigungsmechanismus wirksam schalten kann.

In vorteilhafter Weiterbildung der Erfindung kann der genannte Kupplungsschalter dabei auch noch andere Signale oder Betriebsparameter berücksichtigen und nicht unmittelbar nur bei einer erfassten Bewegung der Bewegungstaste den Übertrager einkuppeln, sondern das Signal des Bewegungssensors sozusagen nur dazu nutzen, um weitere Betriebsparameter des Systems abzufragen, anhand derer dann entschieden wird, ob der Übertrager tatsächlich eingekuppelt wird oder nicht. Insbesondere kann der genannte Kupplungsschalter von einer Steuervorrichtung in Abhängigkeit einer bereits verabreichten Dosis und/oder in Abhängigkeit einer zulässigen Höchstdosis und/oder in Abhängigkeit des Vorliegens eines patientenindividuellen Identifizierungscodes und/oder in Abhängigkeit des Vorliegens eines Freischaltcodes und/oder in Abhängigkeit eines Zeitschaltcodes und/oder in Abhängigkeit des Vorliegens eines RFID-Signals freigeschalten werden. Insbesondere kann der genannte Kupplungsschalter bzw. die genannte Steuervorrichtung dann, wenn vom Bewegungssensor eine Betätigung der Betätigungstaste gemeldet wird, abfragen, ob ein RFID-Signal vorliegt oder abfragbar ist, welches einen vorbestimmten Freischaltcode enthält. Ist dies der Fall, kann der Kupplungsschalter dann den Übertrager einkuppeln. In ähnlicher Weise kann nach Melden einer Bedientastenbewegung abgefragt werden, ob und/oder in welcher Höhe eine Wirkstoffdosis in einem vergangenen Zeitfenster bereits verabreicht wurde, und/oder ob eine zulässige Höchstdosis bzw. Tageshöchstdosis schon erreicht wurde und/oder ob eine autorisierte Bedienperson - beispielsweise durch Übertragung eines patientenindividuellen Identifizierungscodes - in der Nähe des Geräts ist. Beispielsweise kann hierzu ein Benutzer ein RFID-Armband oder ein RFID-Element, das an anderer Stelle des Körpers getragen oder befestigt werden kann, verwenden, um über eine drahtlose Kommunikationsverbindung anhand des RFID-Element übertragenen Daten zu entscheiden, ob der Übertrager eingekuppelt werden soll. Alternativ oder zusätzlich kann zur Identifizierung des Bedieners auch ein Fingerabdrucksensor vorgesehen sein.

Alternativ oder zusätzlich zu einem solchen elektronischen Signalcode kann zum Schutz vor unbeabsichtigter oder unbedachter Auslösung auch eine mechanische Betätigungssperre vorgesehen sein, die die genannte Betätigungstaste sperrt und erst gelöst werden muss, um ein Bewegen der Betätigungstaste zu ermöglichen. Eine solche Kindersicherung oder Mehrhand- bzw. Mehrfingerbetätigung kann bspw. durch eine oder mehrere zusätzliche Endsperrtasten realisiert sein, die bspw. quer zur Bedientaste nach Art eines Riegels arbeiten können. In Weiterbildung der Erfindung können bspw. am Applikatorgehäuse zwei Sperrbacken vorgesehen sein, die quer zur Betätigungsebene der Bedientaste niederdrückbar bzw. bewegbar gelagert sein können, sodass sie durch Niederdrücken die Bedientaste freigeben und in der nicht-gedrückten Stellung die Bedientaste sperren.

Um ein unnötiges Einschalten des Nasenapplikators bzw. ein unnötiges Arbeiten der Steuervorrichtung zu vermeiden, kann der genannte Kupplungsschalter und/oder der genannte Bewegungssensor eine Ansprechschwelle aufweisen, die kleine Auslenkungen der Bedientaste aus deren Ausgangsstellung unberücksichtigt lässt. Der Kupplungsschalter bzw. die diesem zugeordnete Steuervorrichtung reagiert also erst, wenn die Bedientaste eine bestimmte Wegstrecke zurückgelegt hat, bei deren Überschreiten vernünftigerweise angenommen werden kann, dass es sich um eine willentliche Bedientastenbetätigung handelt. Beispielsweise kann die genannte Ansprechschwelle 20% bis 50% des maximalen Stellwegs betragen, also beispielsweise ein Reagieren des Kupplungsschalters erst dann vorsehen, wenn die Bedientaste beispielsweise ein Viertel ihres vorgesehenen Wegs bewegt wird. Je nach Stellweg und Ausbildung der Bedientaste kann die genannte Ansprechschwelle jedoch auch kleiner oder größer sein.

Um Fehlbedienungen sicher zu vermeiden, kann ein Einkuppeln des Übertragers auch erst bei einer Mehrfachbetätigung der Bedientaste vorgenommen werden bzw. kann der Kupplungsschalter wegstreckenabhängig und zyklusabhängig eine Bedientastenbetätigung in eine Einkuppelbewegung umsetzen. Insbesondere kann der Kupplungsschalter in Abhängigkeit des Bewegungssensors derart arbeitend ausgebildet sein, dass der Übertrager erst eingekuppelt wird, wenn die Bedientaste nach einem ersten Tastenhub, der im Freigang erfolgen kann, zurück in die Ausgangsstellung bewegt wird, sodass dann in einem zweiten oder weiteren Tastenhub die Betätigungstasten-Stellbewegung auf den Abgabedosierer übertragen wird. Hierdurch kann - bei Vorliegen ggfs. nötiger, weiterer Freischaltsignale wie dem zuvor genannten Freischaltcode - eine Betätigung des Abgabedosierers nach Art eines Doppelklicks oder Mehrfachklicks einer Computermaus initialisiert werden. Ein erstes Niederdrücken oder ggfs. auch ein mehrfaches, beispielsweise zweifaches oder dreifaches Niederdrücken im Freigang bewirkt ein Aufwecken bzw. Scharfstellen des Betätigungsmechanismus und veranlasst den Kupplungsschalter, den Übertrager einzukuppeln und eine Wirkverbindung zwischen Bedientaste und Abgabedosierer herzustellen, während dann ein folgender oder weiterer Betätigungshub der Bedientaste in eine Betätigung des Abgabedosierers umgesetzt wird. Der Kupplungsschalter kann also derart ausgebildet sein, dass er den Übertrager erst dann scharfstellt, wenn eine vorbestimmte Anzahl von Bedientastenbetätigungen im Freigang erfasst wurde, wobei dies im einfachen Fall nur eine freigängige Leerbetätigung zu sein braucht, um dann gleich bei der zweiten Bedientastenbetätigung die Betätigungsstellbewegung in einen Dosierhub umzusetzen.

Um zu verhindern, dass der Abgabedosierer nach Herstellen der Wirkverbindung zwischen Bedientaste und Abgabedosierer unbeabsichtigt oder unberechtigterweise mehrfach betätigt wird, beispielsweise durch rasches Mehrfachbetätigen der Bedientaste, kann der genannte Kupplungsschalter eine Zeitschaltung zum zeitlich nur begrenzten Einkuppeln des Übertragers aufweisen, sodass der Übertrager nach Ablauf einer vorbestimmten Zeitspanne, die auch sehr kurz sein kann, wieder auskuppelt.

Alternativ oder zusätzlich kann der Kupplungsschalter auch eine Dosierhubschaltung zum Einkuppeln des Übertragers für nur einen einzelnen Dosierhub aufweisen. Eine solche Dosierhubschaltung kann grundsätzlich in verschiedener Weise realisiert sein, beispielsweise durch Erfassung einer Rückstellbewegung der Bedientaste durch den genannten Bewegungssensor, insbesondere dergestalt, dass dann, wenn die Bedientaste nach einem Niederdrücken wieder in ihre Ausgangsstellung zurückfährt, der Kupplungsschalter deaktiviert bzw. der Übertrager wieder ausgekuppelt wird.

Alternativ oder zusätzlich kann der Übertrager auch in seine ausgekuppelte Stellung vorgespannt sein, beispielsweise mittels einer Feder- oder Vorspannvorrichtung oder auch nur schwerkraftbetätigt, wobei der Übertrager in seiner Kupplungsstellung nur kraft- oder reibschlüssig oder formschlüssig dadurch gehalten wird, dass die Bedientaste gegen den Übertrager gedrückt oder gezogen wird. Lässt der Eingriffsdruck zwischen Übertrager und Bedientaste nach, beispielsweise weil die Bedientaste losgelassen oder zurückgestellt wird, kann die Vorspannkraft den Eingriff zwischen Übertrager und Bedientaste lösen, sodass der Übertrager in seine ausgekuppelte Stellung zurückfällt.

Der genannte Übertrager kann Teil eines mehrgliedrigen und/oder mehrstückigen und/oder mehrgelenkigen Übertragermechanismus und/oder -strangs sein, der in Weiterbildung der Erfindung rein mechanisch ausgebildet sein kann und/oder eine Betätigung des Wirkstoffförderers bzw. Abgabedosierers durch Muskelkraft erreichen kann. Grundsätzlich wäre es jedoch ebenfalls möglich, fremdenergiebetätigbare Stellaktoren unterstützend oder auch die Förderung vollständig bewirkend vorzusehen, sodass die Stellbewegung der Betätigungstaste im Wesentlichen nur dem in Betrieb setzen eines solchen fremdenergiebetätigten Stellaktors und/oder dem Auslösen eines zuvor motorisch oder durch Fremdenergie gespannten Stell- bzw. Treibmechanismus dient. Um jedoch keine großen Batterien zu benötigen, kann eine im Wesentlichen vollständige Muskelkraftbetätigung des Abgabedosierers vorgesehen sein.

Der Abgabedosierer bzw. dessen Wirkstoffförderer kann dabei grundsätzlich direkt durch den Übertrager von der Bedientaste her betätigt bzw. betrieben werden. Ist beispielsweise in der eingangs genannten Weise eine Wirkstoff- bzw. Nasenspray-Kartusche mit ihrem Wirkstoffbehältnis verschieblich im Applikatorgehäuse aufgenommen, kann der Übertrager die Stellbewegung der Bedientaste direkt und unmittelbar in eine Stell- bzw. Verschiebebewegung des Wirkstoffbehältnis umsetzen, um dadurch die Wirkstoffkartusche zu betätigen. Hierdurch kann ein einfacher Aufbau des Wirk- bzw. Antriebsstrangs zwischen Bedientaste und Dosierförderer erzielt werden.

Nichtsdestotrotz kann hierbei, um eine einfache Betätigung mit wenig Kraft zu ermöglichen, ein ggfs. mehrstückiger oder mehrgliedriger Hebelmechanismus vorgesehen sein, der die Bedientastenbewegung in die gewünschte Kartuschenbewegung umsetzt, wobei der genannte Übertrager Teil dieses Hebelmechanismus sein kann.

Alternativ zu einer solchen unmittelbaren Betätigung kann nach einem weiteren Aspekt der vorliegenden Erfindung jedoch auch eine nur mittelbare bzw. indirekte Betätigung des Dosierförderers bzw. Abgabedosierers durch die Bedientaste vorgesehen sein. Dabei kann der Abgabedosierer einen spann- und auslösbaren Vorspannmechanismus zum Betätigen des Dosierförderers mit einer definierten Vorspannkraft aufweisen, wobei der Vorspannmechanismus durch den Übertrager gegen einen Rückhalter spannbar ausgebildet und der Rückhalter durch den Übertrager lösbar ausgebildet ist, sodass der Vorspannmechanismus von dem Übertrager sowohl spannbar als auch lösbar ist. Eine solche indirekte Betätigung des Abgabedosierers durch Muskelkraft, deren Betätigung der Bedientaste in das Spannen des Vorspannmechanismus umgesetzt wird, kann in Verbindung mit der zuvor erläuterten Ein- und Auskuppelbarkeit des Übertragers vorgesehen, jedoch auch unabhängig hiervon vorteilhaft sein, um eine präzise, exakte reproduzierbare Dosierung des Wirkstoffs zu ermöglichen. Insbesondere kann durch einen solchen Vorspannmechanismus, der von der Bedientaste über den Übertrager ausgelöst wird, ein definiertes Sprühbild erzeugt werden, das die genannte Tröpfchengröße besitzt bzw. den Wirkstoff mit der gewünschten Feinheit zerstäubt und einen immer gleichen Sprühkegel beinhaltet. Der gespannte Vorspannmechanismus erzeugt unabhängig davon, wie schnell oder wie kraftvoll der Betätiger niedergedrückt wird, eine definierte Stellkraft und -geschwindigkeit, die in dem gewünschten Sprühbild mündet. Da der Dosierförderer nicht unmittelbar von der Geschwindigkeit oder der Stärke der Betätigungskraft abhängt, sondern von der Vorspannkraft des Vorspannmechanismus getrieben wird, kann mit jedem Hub die exakt gewünschte Dosiermenge ausgegeben und das gewünschte Sprühbild erzielt werden. Gleichzeitig kann eine energiesparende Gestaltung des Applikators erzielt werden, da auf fremdenergiebetätigte Stellaktoren und Pumpen und dergleichen verzichtet werden kann.

Die Kombination eines solchen Vorspannmechanismus mit einem Betätiger, der den Vorspannmechanismus auslöst, kann das gewünschte, immer gleichbleibende bzw. reproduzierbare Sprühbild auch schon dann erzeugen, wenn der Betätiger nur zum Auslösen des Vorspannmechanismus vorgesehen ist und der Vorspannmechanismus über einen fremdenergiebetätigbaren Spannaktor gespannt werden kann, beispielsweise in Form eines batteriegetriebenen Motors, der beispielsweise über eine Spindel eine Stellfeder spannen kann. Gegebenenfalls kann der Betätiger und/oder der damit verbundene Übertrager den fremdenergiebetätigbaren Spannaktor starten bzw. in Gang setzen, beispielsweise indem mit einem ersten Niederdrücken der Spannmotor gestartet wird, der dann den Vorspannmechanismus spannt, und ein zweites Niederdrücken den gespannten Vorspannmechanismus auslöst. Mit anderen Worten muss der Vorspannmechanismus nicht zwangsweise durch Muskelkraft gespannt werden. Gleichwohl kann eine besonders kleinbauende, leichte Ausbildung ohne Abhängigkeit von der Lebensdauer von Batterien erzielt werden, wenn der genannte Vorspannmechanismus durch die Bedientaste und den damit verbundenen Übertrager und somit durch Muskelkraft spannbar ist.

In Weiterbildung der Erfindung kann ein Zusammenspiel des Rückhalters und des Spanners des Vorspannmechanismus mit unterschiedlichen Abschnitten des Stellwegs des Übertragers bzw. ein zeitlich abgestuftes Zusammenspiel zwischen Übertrager und Rückhalter sowie Übertrager und Spanner vorgesehen sein, insbesondere derart, dass der Übertrager zunächst nur den Spanner betätigt und den Rückhalter erst dann löst, wenn der Vorspannmechanismus vollständig gespannt ist.

Insbesondere kann der Rückhalter einem anderen Abschnitt des Stellwegs des Übertragers zugeordnet sein als der genannte Spanner. Vorteilhafterweise ist der Rückhalter relativ zum Stellweg des Übertragers derart angeordnet, dass der Rücksteiler erst in einem Endabschnitt des Stellwegs des Übertragers mit dem Übertrager in Eingriff gerät und erst dann ausgelöst wird, wenn der Vorspannmechanismus vollständig gespannt ist.

Hierdurch erhält der Übertrager eine Doppelfunktion, nämlich einerseits Spannen und andererseits Auslösen. Vorteilhafterweise kann das Spannen und das Auslösen des Vorspannmechanismus mit nur einer Betätigung, beispielsweise einem einfachen Niederdrücken der Betätigungstaste erledigt werden. Der Applikatorbediener braucht nicht verschiedene Tasten bedienen oder die Bedientaste mehrfach niederdrücken.

Vorteilhafterweise kann der genannte Vorspannmechanismus eine Hebevorrichtung beispielsweise in Form eines Kniehebels aufweisen, die bzw. der die Bewegung des Übertragers unter einem entsprechenden Hebelverhältnis in eine Spannbewegung einer Federeinrichtung umsetzt. Die genannte Federeinrichtung kann dabei vorteilhafterweise eine mechanische Feder beispielsweise in Form einer Spiralfeder sein oder auch eine Gas- oder Fluiddruckfedereinrichtung umfassen. Durch Verwendung eines solchen Hebelmechanismus kann eine einfache Handhabung mit niedrigen Kräften auch von geschwächten Patienten erzielt werden.

Wird in der zuvor genannten Weise mittels einer Dosiererfassungseinrichtung die abgegebene Wirkstoffmenge erfasst, kann der Nasenapplikator sicherstellen, dass die für einen Patienten verträgliche Höchstdosis eingehalten bzw. nicht überschritten wird, beispielsweise dadurch, dass die Steuervorrichtung den Kupplungsschalter dann sperrt, oder den Übertrager dann außer Eingriff hält, wenn die zulässige Höchstdosis erreicht ist.

Dabei kann der Nasenapplikator hinsichtlich der abgebbaren Wirkstoffhöchstmenge individuell konfiguriert werden, wobei einerseits eine Höchstmenge pro Zeit, beispielsweise in Form von Mikrogramm/Stunde, und/oder andererseits auch absolute Höchstmengen, beispielsweise bei nicht ohne Weiteres abbaubaren Wirkstoffen, vorgegeben werden können, bei deren Erreichen die Sperrvorrichtung zum Sperren des Abgabedosierers jeweils aktiviert wird, so dass der Abgabedosierer nur die jeweils individuell vorgegebene Wirkstoffhöchstmenge abgeben kann. Die genannten zulässigen Wirkstoffhöchstmengen können durch Eingeben entsprechender Steuerungsdaten in den genannten Patientendatenspeicher variabel vorgegeben werden. In Weiterbildung der Erfindung können die Applikationsmengen bzw. - höchstmengen, die von Abgabedosierer abgegeben werden, beispielsweise in Abhängigkeit des Patientengewichts oder dessen Opioid-Gewohnheiten gesteuert werden.

Die patientenindividuelle Konfigurierung kann hierbei grundsätzlich in verschiedener Art und Weise erfolgen. Beispielsweise kann der Nasenapplikator bzw. dessen Steuervorrichtung und/oder deren Patientendatenspeicher über eine Datenübertragungsschnittstelle an einen übergeordneten, externen Rechner angeschlossen werden, von dem aus die notwendigen Steuerungsdaten für den jeweiligen Patienten bzw. Anwender eingespielt werden können.

Alternativ oder zusätzlich zu der Konfigurierbarkeit über eine externe Datenübertragungsschnittstelle kann auch der Nasenapplikator selbst mit geeigneten Eingabemitteln versehen sein, die die Eingabe der patientenindividuellen Steuerungsdaten in die Steuervorrichtung bzw. deren Patientendatenspeicher ermöglicht, beispielsweise in Form einer Tastatur und/oder eines Touchscreens, entweder direkt am Gehäuse oder in ansteckbarer Form beispielsweise nach Art einer externen PC-Tastatur.

Die vorliegende Erfindung wird nachfolgend anhand eines bevorzugten Ausführungsbeispiels und zugehöriger Zeichnungen näher erläutert. In den Zeichnungen zeigen:
- Fig. 1:: eine schematische Darstellung eines Nasenapplikators nach einer vorteilhaften Ausführung der Erfindung in einer Ausgangsstellung,
- Fig. 2:: eine schematische Darstellung des Nasenapplikators, die das Zusammenspiel mit einem RFID-Armband zeigt,
- Fig. 3:: eine ausschnittsweise, schematische Darstellung des Betätigungsmechanismus zur Betätigung der Wirkstoffkartusche in einer Schnittansicht des Nasenapplikators, wobei der von der Betätigungstaste zu betätigende Übertrager in einer ausgekuppelten Ausgangsstellung und die Vorspannvorrichtung ungespannt gezeigt ist,
- Fig. 4:: eine schematische Schnittansicht des Betätigungsmechanismus ähnlich Figur 3, wobei die Betätigungstaste in einem Aktivier- oder Aufwachmodus im Freigang niedergedrückt wurde und einen Bewegungssensor betätigt hat,
- Fig. 5:: eine schematische Schnittansicht des Betätigungsmechanismus ähnlich Figuren 3 und 4, wobei die Betätigungstaste vor einem zweiten Betätigungshub steht und der Übertrager in seine Einkuppelstellung verfahren wurde, sodass bei weiterem Niederdrücken der Betätigungstaste der Federmechanismus der Vorspannvorrichtung spannbar ist,
- Fig. 6:: eine schematische Schnittansicht des Betätigungsmechanismus ähnlich den Figuren 3 bis 5, wobei der Übertrager immer noch in seiner Einkuppelstellung ist und durch weiteres Niederdrücken der Betätigungstaste der Kniehebelmechanismus die Vorspannvorrichtung gespannt hat, wobei der Übertrager gerade dabei ist, den Rückhalter der Vorspannvorrichtung auszulösen, und
- Fig. 7:: eine schematische Schnittansicht des Betätigungsmechanismus ähnlich den Figuren 3 bis 6, wobei der Rückhalter der Vorspannvorrichtung ausgelöst ist und der Übertrager wieder in eine ausgekuppelte Stellung verbracht ist.

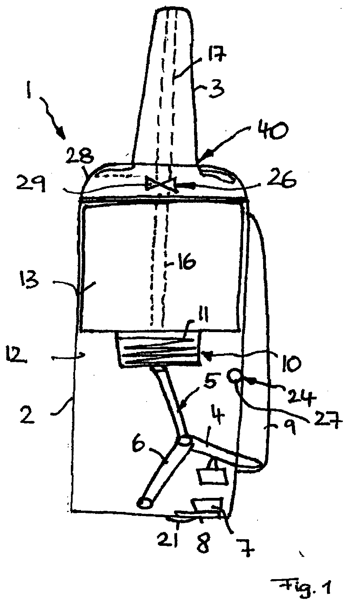

Wie Fig. 1 zeigt, kann der Nasenapplikator 1 ein längliches, näherungsweise zylindrisches Applikatorgehäuse 2 umfassen, von welchem stirnseitig ein Abgabedorn 3 zum Einführen in eine Nasenhöhle abgeht. Das Gehäuse 2 kann ein Handteil zum Greifen des Nasenapplikators 1 bilden und/oder in seinem Inneren eine Aufnahmekammer 12 bilden und darin einen Wirkstoffbehälter 13 auswechselbar aufnehmen, der an den Abgabedorn 3 des Nasenapplikators 1 anschließbar ist. Um den Wirkstoffbehälter 13 wechseln zu können, kann das Gehäuse 2 beispielsweise durch einen Deckel 28 geöffnet, der Wirkstoffbehälter 13 eingesetzt und dann der Deckel 28 wieder geschlossen werden.

Vorteilhafterweise kann der Wirkstoffbehälter 13 zusammen mit dem Abgabedorn 3 eine an sich bekannte Nasenspray- bzw. Wirkstoffkartusche bilden, bei der der Wirkstoffbehälter 13 auf den Abgabedorn 3 zudrückbar ist, um durch diese Hub- bzw. Stauchbewegung den Wirkstoff auszubringen. Wie Fig. 1 zeigt, kann die gesamte Wirkstoffkartusche in das Applikatorgehäuse 2 eingesetzt werden, wenn der Deckel 28 entfernt ist. Der genannte Deckel 28 wird dabei über den Abgabedorn 3 geschoben und hält dessen Flügel dann fest, wenn der Deckel 28 wieder geschlossen ist. Der Wirkstoffbehälter 13 ist im Applikatorgehäuse 2 verschieblich aufgenommen, sodass die Funktion der Wirkstoffkartusche erhalten bleibt.

Die Förderung des Wirkstoffs aus dem eingesetzten Wirkstoffbehälter 13 erfolgt vorteilhafterweise durch einen Förderaktor 26, der beispielsweise zwischen Abgabedorn 3 und Wirkstoffbehälter 13 vorgesehen sein kann, mittels derer der Wirkstoff aus dem Wirkstoffbehälter 13 ausgetrieben wird. Der Wirkstoff kann dabei über eine Kanüle 16, die sich in dem Wirkstoffbehälter 13 erstreckt, über eine Pumpe 29 gefördert werden, von welcher aus der Wirkstoff durch eine Leitung 17 durch den Nasendorn 3 hindurch weitergefördert und an der stirnseitigen Öffnung des Abgabedorns 3 abgegeben wird. Wie zuvor erläutert, kann die genannte Pumpe 29 in die vorkonfigurierte Wirkstoffkartusche umfassend den Wirkstoffbehälter 13 und den Abgabedorn 3 integriert sein.

Zum Dosieren der Wirkstoffmenge kann der genannte Abgabedosierer 4 in Form der Wirkstoffkartusche gemäß Fig. 1 von einer Betätigungstaste 9 am Gehäuse 2 betätigt werden.

Die genannte Betätigungstaste 9 kann beispielsweise ein umfangsseitig am Applikatorgehäuse 2 angeordneter Betätigungshebel sein, der in radialer Richtung in das Gehäuse eindrückbar bzw. darauf zubewegbar ist. Eine solche Betätigungsstellbewegung der Betätigungstaste 9 wird über einen Übertrager 4 auf den Abgabedosierer übertragen, wobei insbesondere der Wirkstoffbehälter 13 axial in Längsrichtung des Applikatorgehäuses 2 auf den Abgabedorn 3 zu verschoben werden kann, um hierdurch eine Dosierhub auszuführen. Wie Fig. 1 andeutet, kann die Bewegung des Übertragers 4 mittelbar auf den Abgabedosierer 3 übertragen werden, insbesondere mittels eines Hebelmechanismus, der durch ein Hebelverhältnis die Stellbewegung umsetzt. Ein solcher Hebelmechanismus 5 kann einen am Applikatorgehäuse 2 abgestützten Kniehebel 6 umfassen, der durch eine Bewegung des Übertragers 4 gestreckt werden kann. Diese Streckbewegung des Kniehebels 6 kann wiederum in eine Spannbewegung einer Vorspannvorrichtung 10 umgesetzt werden, die beispielsweise eine Federeinrichtung 11 in Form einer mechanischen Feder wie beispielsweise einer Spiralfeder aufweisen kann. Die genannte Vorspannvorrichtung 10 kann dann ausgelöst werden, um den Abgabedosierer in der genannten Weise zu betätigen.

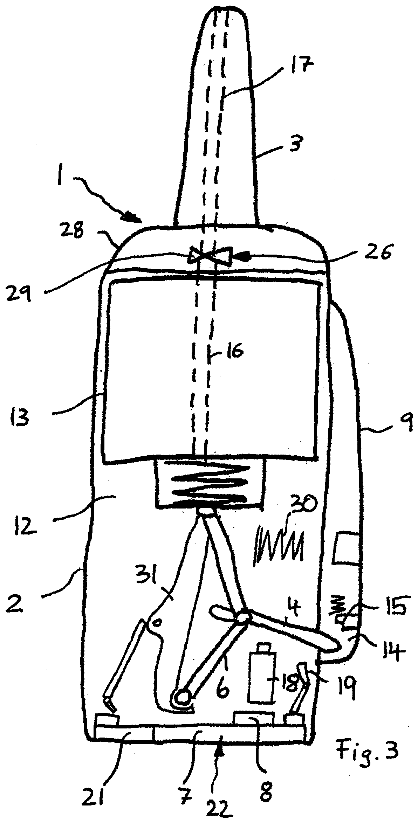

Die Ausbildung des Betätigungsmechanismus und die genannte Betätigung über die Vorspannvorrichtung 10 wird nachfolgend näher anhand der Figur 3 bis 7 erläutert: Wie Fig. 3 zeigt, kann der genannte Übertrager 4 quer zur Längsachse des Applikatorgehäuses 2 beweglich gelagert sein, um durch die genannte Betätigungstaste 9 bewegt werden zu können. Insbesondere kann der genannte Übertrager 4, der beispielsweise ein stabförmiges, längliches Element bilden kann, an dem vorgenannten Kniehebel 6 angelenkt sein, beispielsweise im Bereich von dessen Kniegelenk.

Mit einem anderen Endabschnitt kann der Übertrager 4 sich im Bereich der Betätigungstaste 9 erstrecken, wobei die Betätigungstaste 9 eine Ausnehmung 14 aufweisen kann, in die der Übertrager 4 einfahren kann, um der Bedientaste 9 gegenüber dem Übertrager 4 einen Freilauf bzw. eine Freigängigkeit zu geben.

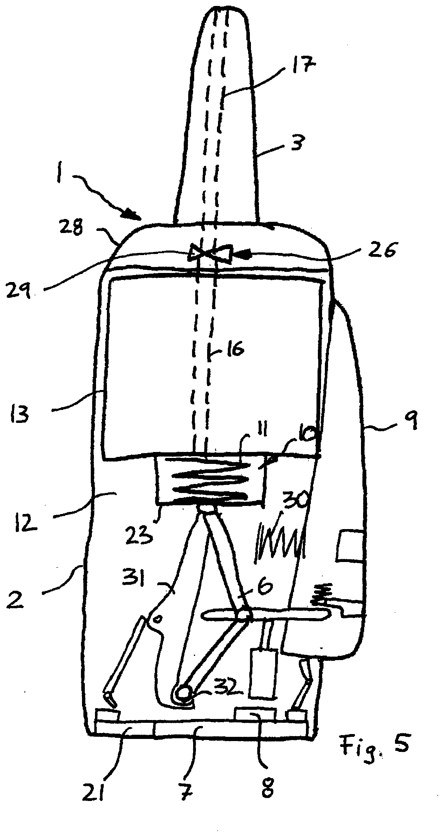

Andererseits kann die Bedientaste 9 in Nachbarschaft zu der genannten Ausnehmung 14 eine Eingriffskontur 15 aufweisen, an der sich der Übertrager 4 abstützen kann, um eine Stellbewegung der Bedientaste 9 aufzunehmen und umzusetzen. Wie insbesondere die Figur 5 zeigt, kann die genannte Eingriffskontur 15 einen Anschlag bilden und/oder eine Abstützfläche aufweisen, an der sich der Übertrager 4 in Richtung seiner Soll-Bewegungsrichtung abstützen kann. Insbesondere kann sich die genannte Eingriffskontur 15 näherungsweise quer zu der vorbestimmten Bewegungsrichtung der Bedientaste 9 erstrecken.

Vorteilhafterweise kann die genannte Eingriffskontur 15 einen Absatz bilden, dessen einer Schenkel der Führung des Übertragers 4 dient und dessen anderer Schenkel der Abstützung in Soll-Bewegungsrichtung dient.

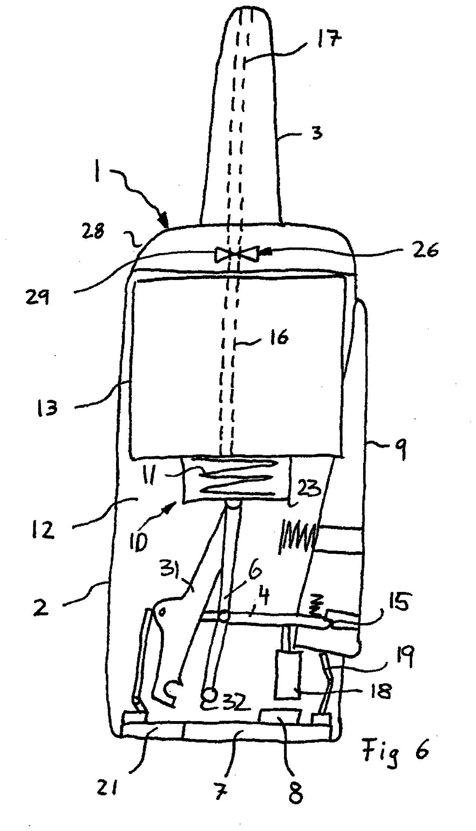

Um den Übertrager 4 zwischen seiner in Figur 3 gezeigten, ausgekuppelten Ausgangsstellung, in der der Übertrager 4 freigängig in die Aufnahmetasche 14 der Bedientaste 9 einfahren kann, und der eingekuppelteten Wirkstellung bzw. Übertragerstellung, wie sie die Figuren 5 und 6 zeigen und bei der der Übertrager 4 mit dem Bedienhebel 9 in Eingriff steht, hin und her bewegen zu können, kann ein Stellaktor 18 beispielsweise in Form eines Elektromagneten oder eines anderen geeigneten, fremdenergiebetätigbaren Aktors vorgesehen sein, der von einer Steuervorrichtung 7 her angesteuert werden kann.

Insbesondere kann der Übertrager 4 von seiner ausgekuppelten Stellung in seine eingekuppelte Stellung verbracht werden, indem die Bedientaste 9 einmal niedergedrückt bzw. betätigt wird. Eine solche zunächst freigängige Bewegung der Bedientaste 9 wird von einem Bewegungssensor 19 erfasst, der ebenfalls im Applikatorgehäuse 2 angeordnet sein kann. Ein solcher Bewegungssensor 19 kann unterschiedlich ausgebildet sein, beispielsweise berührungslos arbeiten. Hierbei sind verschiedene Arbeitsprinzipien wie Lichtschranke und dergleichen möglich. Vorteilhafterweise kann aber auch ein taktil arbeitender Bewegungssensor 19 vorgesehen sein, der mit der Bedientaste 9 in Berührung gerät, wenn die Bedientaste 9 niedergedrückt wird.

Zeigt der Bewegungssensor 19 eine Bewegung der Bedientaste 9 an, kann der genannte Stellaktor 18 den Übertrager 4 in die eingekuppelte Stellung verbringen, wie dies Figur 5 zeigt. Bevor der Stellaktor 18 jedoch tatsächlich betätigt wird, können weitere Betriebsparameter abgefragt werden, bevor das System insgesamt tatsächlich scharfgestellt wird. Insbesondere kann das Stellsignal des Bewegungssensors 19 zunächst nur dazu verwendet werden, über die zuvor genannte elektronische Steuervorrichtung 7 Dosierungsdaten und Patientendaten wie Identfizierungscode, persönliche zulässige Dosis und dergleichen abzufragen.

Diese elektronische Steuervorrichtung 7 kann vorteilhafterweise ebenfalls im Inneren des Gehäuses 2 untergebracht sein und umfasst einen Patientendatenspeicher 8, aus dem die elektronische Steuervorrichtung 7 für einen jeweiligen Patienten individuell vorgebbare Steuerungsdaten wie Höchstdosis, Einmaldosis oder Tagesdosis auslesen kann. Die genannte Steuervorrichtung 7 und der genannte Patientendatenspeicher 8 können beispielsweise von einem Mikrocontroller und zugehörigen Schaltungsbausteine wie beispielsweise einem Speicherelement gebildet sein, wobei der Patientendatenspeicher 8 beispielsweise an dem auswechselbaren Wirkstoffbehälter 13 oder am Applikatorgehäuse 2 angebracht oder mit einem daran angebrachten Speicherbaustein verbindbar sein kann.

Die Steuervorrichtung 7 kann das Einkuppeln des Übertragers 4 sperren, bzw. außer Betrieb setzen, beispielsweise dann, wenn eine Höchstdosis erreicht ist oder ein Freischaltcode nicht vorliegt. Zur Erfassung der tatsächlichen Abgabemenge umfasst der Nasenapplikator 1 eine Dosiserfassungseinrichtung, die beispielsweise in die Steuervorrichtung 7 integriert sein kann und die Betätigungen des Abgabedorns zählt und/oder die Länge der Betätigungen bestimmt.

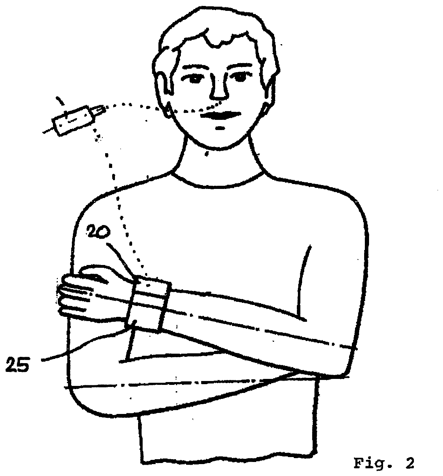

Alternativ oder zusätzlich kann, wie dies Fig. 2 verdeutlicht, der Nasenapplikator 1 bzw. dessen Steuervorrichtung 7 auch mit einem RFID-Element 20 kommunizieren, welches der Patient beispielsweise mittels eines Armbands oder eines anderes Körperbefestigungsmittels 25 am Körper trägt. In dem genannten RFID-Element 20 können einerseits patientenindividuelle Steuerungsdaten beispielsweise betreffend die zulässige Höchstdosis abgelegt sein, und/oder ein Freischaltcode, welcher den Nasenapplikator 1 überhaupt erst freischaltet bzw. die genannte Sperrvorrichtung 6 entriegelt. Hierzu kann die Steuervorrichtung 7 mittels einer RFID-Sende-/ Empfangs-einrichtung 21 mit dem genannten RFID-Element 20 kommunizieren, wenn der Nasenapplikator nahe genug bei dem Patienten ist.

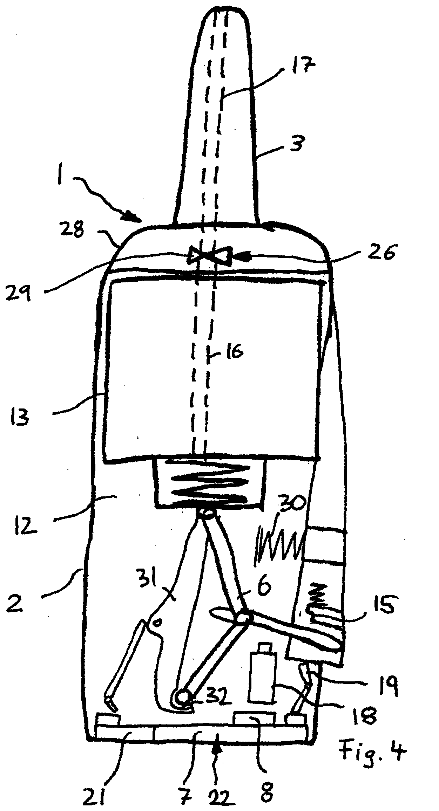

Ergibt die Datenabfrage der Steuervorrichtung 7, dass der Nasenapplikator freigeschaltet werden kann, kann der Kupplungsschalter 22 das Einkuppeln des Übertragers 4 veranlassen. Wie Fig. 4 zeigt, ist dies zunächst noch nicht möglich, wenn die Bedientaste 9 noch vollständig eingedrückt ist bzw. sich noch im ersten freigängigen Betätigungshub befindet. Wird jedoch, wie Fig. 5 zeigt, die Betätigungstaste nach dem Initialisieren des Systems losgelassen, sodass sie zurück in ihre Ausgangsstellung fahren kann, was durch eine Federeinrichtung 30 unterstützt werden kann, kann der genannte Kupplungsschalter 22 den Übertrager 4 in seine Einkuppelstellung verbringen. Genauer gesagt kann der Stellaktor 18 in Form des Elektromagneten den Übertrager 4 in seiner Eingriffsstellung verschwenken, in der der Übertrager 4 an der stufenförmig ausgebildeten Eingriffskontur 15 der Bedientaste 9 anliegt, vgl. Fig. 5. Wird die Bedientaste 9 sodann betätigt bzw. niedergedrückt, gelangt der Übertrager 4 mit seinem stirnseitigen Ende gegen die sich quer zur Betätigungsrichtung erstreckende Flanke der Eingriffskontur 15, sodass der Übertrager 4 reibschlüssig und/oder formschlüssig - durch die Betätigungskraft auf die Bedientaste 9 - in der Einkuppelstellung gehalten wird. Hierbei kann der Stellaktor 18 zeitgeschaltet oder auch weggesteuert - beispielsweise durch ein Signal des Bewegungssensors 19 - bereits wieder zurückgefahren werden, auch wenn dies Fig. 6 nicht zeigt.

Wird die Bedientaste 9 bei eingekuppelter Stellung des Übertragers 4 weiter niedergedrückt, verfährt der Übertrager 4 entsprechend der Stellbewegung der Betätigungstaste 9 quer zur Längsrichtung des Applikatorgehäuses 2. Dementsprechend wird der Kniehebel 6 gestreckt, vgl. vergleichend die Figuren 5 und 6.

Diese Streckbewegung des Kniehebels 6 spannt über den Spanner 23 die Federeinrichtung 11 der Vorspannvorrichtung 10, die durch den Rückhalter 31 noch zurückgehalten wird, sodass die Kartusche noch nicht betätigt wird. Der genannte Rückhalter 31 kann hierbei im weiteren Verfahrweg des Übertragers 4 angeordnet sein, sodass bei vollständigem Niederdrücken bzw. Betätigen der Bedientaste 9 in einem Endabschnitt der Bewegung des Übertragers 4 der genannte Übertrager 4 den Rückhalter 31 auslöst, vgl. vergleichend Figuren 5,6 und 7. Insbesondere kann sich der Rückhalter 31 - grob gesprochen - quer zur Bewegungsrichtung des Übertragers 4 und/oder in Längsrichtung des Applikatorgehäuses 2 erstrecken, sodass der Übertrager 4 im Endabschnitt seiner Stellbewegung gegen den Rückhalter 31 drückt und diesen mitnimmt. Hierdurch wird der Rückhalter 31 ausgelöst. Beispielsweise kann sich der Formschluss zu einer Rückhaltenase 32 lösen, an der der Rückhalter 31 ansonsten in Wirkrichtung der Federeinrichtung 11 zurückgehalten wird. Beispielsweise kann der Rückhalter 31 auch federvorgespannt an der genannten Rückhaltenase 32 gehalten sein, sodass er nur durch die Stellkraft des Übertragers 4 auslösen kann.

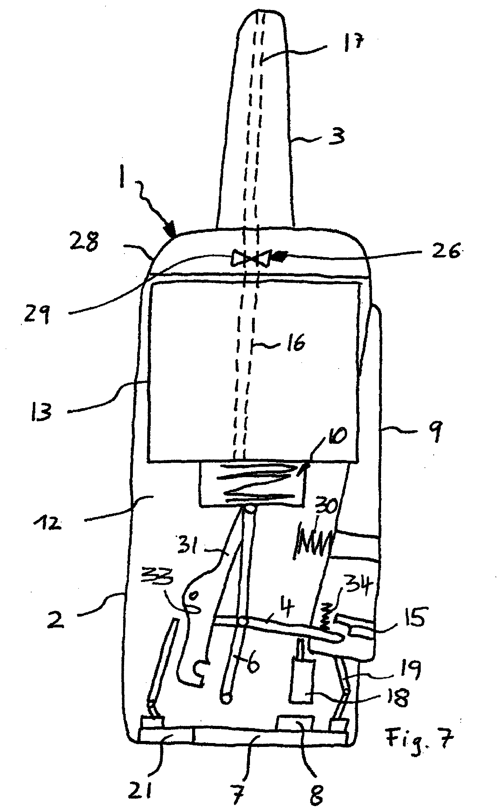

Löst der RücKhalter 31 aus, wie dies Figur 7 zeigt, kann die Federeinrichtung 11 der Vorspannvorrichtung 10 aufgrund ihrer Spannkraft losschnellen und hierdurch den Abgabedosierer betätigen, genauer gesagt den Wirkstoffbehälter 13 der Kartusche auf den Abgabedorn 3 zu bewegen, wodurch ein Dosierhub ausgeführt wird.

Vorteilhafterweise kann der auslösende Rückhalter 31 bei der Stellbewegung der Feder 11 gleichzeitig den Übertrager 4 in seine ausgekuppelte Stellung zurückbewegen, wenn er in diese ausgekuppelte Stellung nicht bereits von selbst zurückgegangen ist. Beispielsweise kann der Rückhalter 31 eine Rückstellnase 33 aufweisen, die bei Bewegen des Rückhalters 31 in Richtung der Federkraft der Federeinrichtung 11 quer zur Stellbewegung des Übertragers 4 auf diesen aufwirkt, bzw. mit dem Übertrager 4 in Eingriff gerät, wodurch der Übertrager 4 in seine ausgekuppelte Stellung zurückbewegt wird.

Eine solche Rückstellbewegung des Übertragers 4 kann auch durch eine separate Rückstellfeder 34 unterstützt und/oder ausgelöst und/oder bewirkt werden, vgl. Figur 7.

Bei Loslassen der Betätigungstaste 9 bewegt sich das System insgesamt wieder zurück in seine Ausgangsstellung, wie sie in Figur 3 gezeigt ist.

Um den Applikator weiter vor unbedachter Betätigung bspw. durch Kleinkinder zu schützen, kann alternativ oder zusätzlich zu der vorgenannten RFID-Freischalteinrichtung optional auch eine mechanische Betätigungssperre 24 vorgesehen sein, die in ihrer Ausgangsstellung die Bedientaste 9 sperrt und erst gelöst werden muss, um ein Niederdrücken der Bedientaste 9 zu ermöglichen. Eine solche Betätigungssperre 24 bzw. Kindersicherung kann bspw. einen oder mehrere Querriegel 27 am Applikatorgehäuse 2 umfassen, die quer zur Bewegungsebene der Bedientaste 9 verstellbar sind, bspw. durch niederdrückbare Endsperrtasten bzw. -backen, die seitlich aus dem Applikatorgehäuse 2 herausstehen können, vgl. Fig. 1.

Der beschriebene Nasenapplikator kann insbesondere zur Verabreichung von hochwirksamen Opioidschmerzmitteln verwendet werden, wobei der Nasenapplikator grundsätzlich jedoch auch zur Abgabe anderer Medikamente, die unter Kontrolle des Patienten selbst verabreicht werden, eingesetzt werden kann. Dabei kann es sich insbesondere um Substanzen handeln, die unter zumindest einer der folgenden Randbedingungen sinnvoll verabreicht werden: a) Die zu therapierende Erkrankung beziehungsweise Beschwerden/Symptome setzen plötzlich ein und lassen sich nicht mit ausreichender Genauigkeit vorhersehen (beispielsweise Migräneattacken, Übelkeit/Erbrechen, Blutzuckerschwankungen, epileptische Anfälle, extreme Blutdruckschwankungen). b) Die zur Behandlung der oben genannten Symptome eingesetzten Medikamente werden schnell über die Nasenschleimhaut aufgenommen und bewirken eine schnelle Verbesserung der Beschwerden. c) Die zur Behandlung der unter a) genannten Symptome eingesetzten Medikamente beinhalten ein gewisses Gefahrenpotenzial oder weisen starke Nebenwirkungen auf, d.h. ihre Applikation muss in bestimmten Grenzen geregelt werden (beispielsweise können die so genannten "Triptane" schnell und zuverlässig bei Migräneattacken wirken, aber bei einer Überdosierung zu schweren Durchblutungsstörungen führen). d) Die zur Behandlung der unter a) genannten Symptome eingesetzten Medikamente können zur Abhängigkeit/Sucht führen (Beispiele: Opioide, Antiepileptika), so dass im Rahmen einer patientenkontrollierten Applikation eine Zugangskontrolle bzw. Patientenidentifikation erfolgen muss.

## Patentansprüche

1. Nasenapplikator zum nasalen Verabreichen von medizinischen Wirkstoffen, insbesondere Schmerzmitteln, mit einem in einem Applikatorgehäuse (2) angeordneten Wirkstoffspeicher, einem in ein Nasenloch einführbaren Abgabedorn (3) sowie einem Abgabedosierer (40) zum dosierten Abgeben des Wirkstoffs aus dem Wirkstoffbehälter (13) über den Abgabedorn (3), wobei an dem Applikatorgehäuse (2) eine von Hand bewegbare Betätigungstaste (9) zum Betätigen des Abgabedosierers (40) vorgesehen ist, deren Betätigungsstellweg durch einen Übertrager (4) auf den Abgabedosierer (40) übertragbar ist, **dadurch gekennzeichnet, dass** der Übertrager (4) ein- und auskuppelbar ausgebildet ist, sodas bei ausgekuppeltem Übertrager (4) die Betätigungstaste (9) im Freigang ohne Übertragung auf den Abgabedosierer (40) bewegbar ist, wobei ein Bewegungssensor (19) zum Erfassen von Bewegungen der Betätigungstaste (9) und ein Kupplungsschalter (22) zum Einkuppeln des Übertragers (4) in Abhängigkeit eines Signals des Bewegungssensors (19) vorgesehen sind.

2. Nasenapplikator nach dem vorhergehenden Anspruch, wobei der Kupplungsschalter (22) von einer Steuervorrichtung (7) in Abhängigkeit zumindest eines weiteren Steuerungsparameters aus der Parametergruppe umfassend eine verabreichte Dosis, eine zulässige Höchstdosis, einen patienten-individuellen Identifizierungscode, einen Freischaltcode, einen Zeitschaltcode und ein RFID-Signal, freischaltbar ist.

3. Nasenapplikator nach einem der vorhergehenden Ansprüche, wobei der Kupplungsschalter (22) in Abhängigkeit des Bewegungssensors (19) derart arbeitend ausgebildet ist, dass der Übertrager (4) erst eingekuppelt wird, wenn die Bedientaste (9) nach einer vorbestimmten Anzahl von Tastenbetätigungen und/oder Freiganghüben, insbesondere nach einem ersten, freigängigen Tastenhub, zurück in die Ausgangsstellung bewegt wird, sodass in einem weiteren Tastenhub die Betätigungsstellbewegung der Bedientaste (9) auf den Abgabedosierer (40) übertragen wird.

4. Nasenapplikator nach einem der vorhergehenden Ansprüche, wobei der Kupplungsschalter (22) eine Zeitschaltung zum zeitlich nur begrenzten Einkuppeln des Übertragers (4) aufweist.

5. Nasenapplikator nach einem der vorhergehenden Ansprüche, wobei der Kupplungsschalter (22) eine Dosierhubschaltung zum Einkuppeln des Übertragers (4) für nur einen Dosierhub und/oder nur eine Betätigung des Abgabedosierers (40) aufweist.

6. Nasenapplikator nach einem der vorhergehenden Ansprüche, wobei der Kupplungsschalter (22) und/oder der Bewegungssensor (19) eine Ansprechschwelle aufweisen, die kleine Auslenkungen der Bedientaste (9) aus deren Ausgangsstellung unberücksichtigt lässt und ein Einkuppeln des Übertragers (4) nur bei Überschreiten der genannten Anspruchsschwelle veranlasst.

7. Nasenapplikator nach einem der vorhergehenden Ansprüche, wobei der Abgabedosierer (40) einen spann- und auslösbaren Vorspannmechanismus (10) zum Betätigen eines Dosierförderers (26) mit einer definierten Vorspannkraft aufweist, wobei der Vorspannmechanismus (10) gegen einen Rückhalter (31) spannbar ausgebildet und der Rückhalter (31) durch den Übertrager (4) lösbar ist, sodass der gespannte-Vorspannmechanismus (10) von dem Übertrager (4) lösbar ist.

8. Nasenapplikator nach dem vorhergehenden Anspruch, wobei der Vorspannmechanismus (10) durch den Übertrager (4) gegen den Rückhalter (31) spannbar ist, sodass der Vorspannmechanismus (10) von dem Übertrager (4) sowohl spannbar als auch lösbar ist.

9. Nasenapplikator nach dem vorhergehenden Anspruch, wobei der Rückhalter (31) und ein Spanner (36) des Vorspannmechanismus (10) unterschiedlichen Abschnitten des Stellwegs des Übertragers (4) zugeordnet sind, wobei der Rückhalter (31) relativ zum Stellweg des Übertragers (4) derart angeordnet ist, dass der Rückhalter (31) erst in einem Endabschnitt des Stellweg des Übertragers (4) mit dem Übertrager (4) in Eingriff gerät und erst ausgelöst wird, wenn der Vorspannmechanismus (10) vollständig gespannt ist.

10. Nasenapplikator nach einem der beiden vorhergehenden Ansprüche, wobei der Vorspannmechanismus (10) einen Kniehebel (6) aufweist, der von dem Übertrager (4) streckbar ist und eine Federeinrichtung (11) gegen den Rückhalter (31) spannt.

11. Nasenapplikator nach einem der Ansprüche 7 bis 10, wobei ein fremdenergiebetätigter Spannantrieb zum Spannen des Vorspannmechanismus (10) und/oder Unterstützen des Spannens des Vorspannmechanismus (10) vorgesehen ist, wobei der genannte Spannantrieb einen Start-Schalter, der von dem Übertrager (4) betätigbar ist, und/oder einen Zeitschalt-Start-Schalter, der zeitgesteuert arbeitend ausgebildet ist, zum Starten des Spannantriebs aufweist:

12. Nasenapplikator nach einem der vorhergehenden Ansprüche, wobei ein Auskuppelmechanismus zum Auskuppeln des Übertragers (4) bei Abbrechen der Stellbewegung der Bedientaste (9) vor Erreichen der vollständig betätigten Bedientastenstellung vorgesehen ist.

13. Nasenapplikator nach dem vorhergehenden Anspruch, wobei der Übertrager (4) durch eine Vorspannvorrichtung und/oder durch Schwerkraft in die ausgekuppelte Stellung vorgespannt ist und in der eingekuppelten Stellung über einen vorbestimmten Abschnitt des Stellwegs der Bedientaste nur reibschlüssig und/oder formschlüssig durch Anliegen an der Bedientaste (9) gehalten ist.

14. Nasenapplikator nach dem vorhergehenden Anspruch, wobei die Bedientaste (9) eine sich quer zur Bewegungsrichtung der Bedientaste (9) erstreckende Eingriffskontur (15) aufweist, an der der Übertrager (4) in der eingekuppelten Stellung ansteht und reibschlüssig gehalten ist, solange die Bedientaste (9) gegen den Übertrager (4) gedrückt oder gezogen wird.

15. Nasenapplikator nach einem der vorhergehenden Ansprüche, wobei eine mechanische Sperrvorrichtung (24) zum Sperren der Bedientaste (9) in deren unbetätigter Stellung vorgesehen ist, wobei die genannte mechanische Betätigungssperre (24) eine vorzugsweise quer zur Betätigungsebene der Bedientaste (9) bewegbare Endsperrtaste zum Endsperren der mechanischen Betätigungssperre und Freigeben der Bedientaste (9) aufweist.

## Claims

1. A nasal applicator for the nasal administration of medicinal active ingredients, in particular analgesics, comprising an active ingredient store arranged in an applicator housing (2); a dispensing pin (3) which can be introduced into a nostril; and a dispensing meter (40) for a metered dispensing of the active ingredient from the active ingredient container (13) via the dispensing pin (3), wherein an actuating button (9) movable by hand is provided at the applicator housing (2) for actuating the dispensing meter (40) whose actuation adjustment path can be transmitted to the dispensing meter (40) by a transmitter (4), **characterized in that** the transmitter (4) is couplable and decouplable so that, with a decoupled transmitter (4), the actuation button (9) is movable in free travel without transmission to the dispensing meter (40); and wherein a motion sensor (19) is provided for detecting movements of the actuating button (9) and a coupling switch (22) is provided for coupling the transmitter (4) in dependence on a signal of the motion sensor (19).

2. A nasal applicator in accordance with the preceding claim, wherein the coupling switch (22) can be released by a control apparatus (7) in dependence on at least one further control parameter from the parameter group comprising an administered dose, a permitted maximum dose, an identification code individual to the patient, a release code, a time switching code and an RFID signal.

3. A nasal applicator in accordance with one of the preceding claims, wherein the coupling switch (22) is configured as working in dependence on the motion sensor (19) such that the transmitter (4) is only coupled when the actuation button (9) is moved back into the starting position after a predetermined number of button actuations and/or release strokes, in particular after a first, free-travel button stroke, so that the actuation adjustment movement of the actuation button (9) is transmitted to the dispensing meter (40) in a further button stroke.

4. A nasal applicator in accordance with one of the preceding claims, wherein the coupling switch (22) has an interval timer for a temporally only limited coupling of the transmitter (4).

5. A nasal applicator in accordance with one of the preceding claims, wherein the coupling switch (22) has a metering stroke circuit for coupling the transmitter (4) for only one metering stroke and/or only an actuation of the dispensing meter (40).

6. A nasal applicator in accordance with one of the preceding claims, wherein the coupling switch (22) and/or the motion sensor (19) has/have a response threshold that leaves small deflections of the actuation button (9) from its starting position out of consideration and only instigates a coupling of the transmitter (4) on an exceeding of said response threshold.

7. A nasal applicator in accordance with one of the preceding claims, wherein the dispensing meter (40) has a loadable and releasable preload mechanism (10) for actuating a metering conveyor (26) with a defined preload force and wherein the preload mechanism (10) is designed as loadable against a retainer (31) and the retainer (31) is releasable by the transmitter (4) so that the loaded preload mechanism (10) is releasable from the transmitter (4).

8. A nasal applicator in accordance with the preceding claim, wherein the preload mechanism (10) is loadable by the transmitter (4) against the retainer (31) so that the preload mechanism (10) is both loadable and releasable by the transmitter (4).

9. A nasal applicator in accordance with the preceding claim, wherein the retainer (31) and a loader (36) of the preload mechanism (10) are associated with different sections of the adjustment path of the transmitter (4); and wherein the retainer (31) is arranged relative to the adjustment path of the transmitter (4) such that the retainer (31) only comes into engagement with the transmitter (4) in an end section of the adjustment path of the transmitter (4) and is only released when the preload mechanism (10) is completely loaded.

10. A nasal applicator in accordance with one of the two preceding claims, wherein the preload mechanism (10) has a toggle lever (6) that is stretchable by the transmitter (4) and loads a spring device (11) against the retainer (31).

11. A nasal applicator in accordance with one of the claims 7 to 10, wherein a load drive actuable by external energy is provided for loading the preload mechanism (10) and/or for assisting the loading of the preload mechanism (10); and wherein said load drive has a start switch that is actuable by the transmitter (4) and/or that has a time switching start switch that is configured for working with time control for starting the load drive.

12. A nasal applicator in accordance with one of the preceding claims, wherein a decoupling mechanism is provided for decoupling the transmitter (4) on an aborting of the adjustment movement of the actuation button (9) before reaching the completely actuated actuation button position.

13. A nasal applicator in accordance with the preceding claim, wherein the transmitter (4) is preloaded into the decoupled position by a preload apparatus and/or by gravity and is only held in the coupled position over a predetermined section of the adjustment path of the actuation button by friction engagement and/or shape matching by contact at the actuation button (9).

14. A nasal applicator in accordance with the preceding claim, wherein the actuation button (8) has an engagement contour (15) which extends transversely to the direction of movement of the actuation button (9) and which the transmitter (4) contacts and by which it is held with friction engagement in the coupled position as long as the actuation button (9) is pressed against or pulled toward the transmitter (4).

15. A nasal applicator in accordance with one of the preceding claims, wherein a mechanical blocking apparatus (24) is provided for blocking the actuation button (9) in its unactuated position; and wherein said mechanical actuation block (24) has a movable unblocking button, preferably movable transversely to the actuation plane of the actuation button (9), for unblocking the mechanical actuation block and releasing the actuation button (9).

## Revendications

1. Applicateur nasal destiné à l'administration par voie nasale de principes actifs médicaux, en particulier d'analgésiques, comprenant un réservoir de principe actif disposé dans un boîtier d'applicateur (2), un cône de distribution (3) pouvant être introduit dans une narine ainsi qu'un doseur de distribution (40) pour la distribution dosée du principe actif provenant du contenant de principe actif (13) par le biais du cône de distribution (3), un bouton d'actionnement (9) déplaçable à la main pour actionner le doseur de distribution (40) étant prévu sur le boîtier d'applicateur (2), la course d'actionnement dudit bouton d'actionnement pouvant être transmise au doseur de distribution (40) par un organe de transmission (4), **caractérisé en ce que** l'organe de transmission (4) est réalisé de manière à pouvoir être accouplé et désaccouplé, de telle sorte que, lorsque l'organe de transmission (4) est désaccouplé, le bouton d'actionnement (9) peut être déplacé librement sans transmission au doseur de distribution (40), un capteur de déplacement (19) pour détecter des déplacements du bouton d'actionnement (9) et un commutateur de couplage (22) pour accoupler l'organe de transmission (4) en fonction d'un signal du capteur de déplacement (19) étant prévus.

2. Applicateur nasal selon la revendication précédente, dans lequel le commutateur de couplage (22) peut être activé par un dispositif de commande (7) en fonction d'au moins un autre paramètre de commande du groupe de paramètres comprenant une dose administrée, une dose maximale autorisée, un code d'identification spécifique au patient, un code d'activation, un code de temporisation et un signal RFID.

3. Applicateur nasal selon l'une des revendications précédentes, dans lequel le commutateur de couplage (22) est réalisé de manière à fonctionner en fonction du capteur de déplacement (19) de telle manière que l'organe de transmission (4) est accouplé uniquement quand le bouton de commande (9) est ramené dans la position initiale après un nombre prédéfini d'actionnements de bouton et/ou de courses libres, en particulier après une première course de bouton libre, de telle sorte que, lors d'une autre course de bouton, le déplacement d'actionnement du bouton de commande (9) est transmis au doseur de distribution (40).

4. Applicateur nasal selon l'une des revendications précédentes, dans lequel le commutateur de couplage (22) comporte un circuit de minuterie pour l'accouplement uniquement limité dans le temps de l'organe de transmission (4).

5. Applicateur nasal selon l'une des revendications précédentes, dans lequel le commutateur de couplage (22) comporte un circuit de course de dosage pour l'accouplement de l'organe de transmission (4) pour seulement une course de dosage et/ou seulement un actionnement du doseur de distribution (40).

6. Applicateur nasal selon l'une des revendications précédentes, dans lequel le commutateur de couplage (22) et/ou le capteur de déplacement (19) comportent un seuil de réponse, qui ne tient pas compte de petites déviations du bouton de commande (9) hors de sa position initiale et provoque un accouplement de l'organe de transmission (4) uniquement lorsque ledit seuil de réponse est dépassé.

7. Applicateur nasal selon l'une des revendications précédentes, dans lequel le doseur de distribution (40) comporte un mécanisme de précontrainte (10) pouvant être mis en tension et libéré pour actionner un transporteur de dosage (26) avec une force de précontrainte définie, le mécanisme de précontrainte (10) étant réalisé de manière à pouvoir être mis en tension contre un organe de retenue (31) et l'organe de retenue (31) pouvant être libéré par l'organe de transmission (4), de telle sorte que le mécanisme de précontrainte (10) mis en tension peut être libéré par l'organe de transmission (4).

8. Applicateur nasal selon la revendication précédente, dans lequel le mécanisme de précontrainte (10) peut être mis en tension contre l'organe de retenue (31) par l'organe de transmission (4), de telle sorte que le mécanisme de précontrainte (10) peut être aussi bien mis en tension que libéré par l'organe de transmission (4).

9. Applicateur nasal selon la revendication précédente, dans lequel l'organe de retenue (31) et un tendeur (36) du mécanisme de précontrainte (10) sont associés à différentes parties de la course d'actionnement de l'organe de transmission (4), l'organe de retenue (31) étant disposé par rapport à la course d'actionnement de l'organe de transmission (4) de telle manière que l'organe de retenue (31) vient en prise avec l'organe de transmission (4) uniquement dans une partie d'extrémité de la course d'actionnement de l'organe de transmission (4) et est déclenché uniquement quand le mécanisme de précontrainte (10) est entièrement mis en tension.

10. Applicateur nasal selon l'une des deux revendications précédentes, dans lequel le mécanisme de précontrainte (10) comporte un levier coudé (6), qui peut être étiré par l'organe de transmission (4) et qui met en tension un dispositif à ressort (11) contre l'organe de retenue (31).

11. Applicateur nasal selon l'une des revendications 7 à 10, dans lequel un entraînement de mise en tension actionné par une énergie externe pour mettre en tension le mécanisme de précontrainte (10) et/ou soutenir la mise en tension du mécanisme de précontrainte (10) est prévu, ledit entraînement de mise en tension comportant un commutateur de démarrage, qui peut être actionné par l'organe de transmission (4) et/ou un commutateur de démarrage temporisateur, qui est réalisé à fonctionnement commandé par temporisation, pour démarrer l'entraînement de mise en tension.

12. Applicateur nasal selon l'une des revendications précédentes, dans lequel un mécanisme de découplage pour découpler l'organe de transmission (4) lors de l'interruption du déplacement d'actionnement du bouton de commande (9) avant que la position entièrement actionnée du bouton de commande soit atteinte est prévu.

13. Applicateur nasal selon la revendication précédente, dans lequel l'organe de transmission (4) est précontraint dans la position découplée par un dispositif de précontrainte et/ou par la gravité et est maintenu dans la position accouplée sur une partie prédéfinie de la course d'actionnement du bouton de commande uniquement par friction et/ou par complémentarité de formes en s'appliquant contre le bouton de commande (9).

14. Applicateur nasal selon la revendication précédente, dans lequel le bouton de commande (9) comporte un contour de prise (15) s'étendant transversalement à la direction de déplacement du bouton de commande (9), sur lequel repose, maintenu par friction, l'organe de transmission (4) dans la position accouplée tant que le bouton de commande (9) est pressé ou tiré contre l'organe de transmission (4).

15. Applicateur nasal selon l'une des revendications précédentes, dans lequel un dispositif de blocage mécanique (24) pour bloquer le bouton de commande (9) dans sa position non actionnée est prévu, ledit blocage d'actionnement mécanique (24) comportant un bouton de déblocage déplaçable de préférence transversalement au plan d'actionnement du bouton de commande (9) pour débloquer le blocage d'actionnement mécanique et libérer le bouton de commande (9).
